# EUROPEAN PATENT APPLICATION

(11) **EP 2 388 249 A1**
(43) Date of publication of application: **23.11.2011**
(21) Application number: 11177764.5
(22) Date of filing: 27.01.2006
(51) Int. Cl.: C07D 239/50, C07D 239/48, A61K 31/505, A61P 31/18

(54) **HIV inhibiting 2-(4-cyanophenylamino) pyrimidine derivatives**

(30) Priority: 27.01.2005 EP 05100521
(62) Divisional of application: 06707872.5
(71) Applicant: Tibotec Pharmaceuticals, Co Cork (IE)
(72) Inventor: Heeres, Jan, 2350 Vosselaar (BE); Lewi, Paulus Joannes, 2300 Turnhout (BE); Janssen, Paul Adriaan Jan, 2350 Vosselaar (BE); Leemans, Rudolf, 2387 Baarle-Hertog (BE); Moens, Luc Jozef Raphael, 2275 Lille (BE)
(74) Representative: Vervoort, Liesbeth

(57) **Abstract**

HIV replication inhibitors of formula 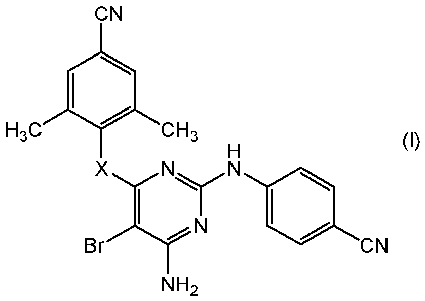 pharmaceutically acceptable addition salts or stereochemically isomeric forms thereof, wherein X is S, SO or SO₂; the preparation of these compounds as well as pharmaceutical compositions comprising these compounds; the use of these compounds for the prevention or the treatment of HIV infection.

## Description

The present invention is concerned with pyrimidine derivatives having HIV (Human Immunodeficiency Virus) replication inhibiting properties, the preparation thereof and pharmaceutical compositions comprising these compounds. The invention also relates to the use of these pyrimidine derivatives in the prevention or treatment of HIV infection.

Resistance of HIV against currently available HIV drugs continues to be a major cause of therapy failure. This has led to the introduction of combination therapy of two or more anti-HIV agents usually having a different activity profile. Significant progress was made by the introduction of "HAART" (Highly Active Anti-Retroviral Therapy), which has resulted in a significant reduction of morbidity and mortality in HIV patient populations treated therewith. HAART involves various combinations of HIV inhibitors selected from different classes such as nucleoside reverse transcriptase inhibitors (NRTIs), non-nucleoside reverse transcriptase inhibitors (NNRTIs) and protease inhibitors (PIs). HAART has been applied succesfully although problems remain. In particular, half of the patients receiving anti-HIV combination therapy do not respond fully to the treatment, mainly because of resistance of the virus to one or more drugs used. Moreover, it has been shown that resistant virus is carried over to newly infected individuals, resulting in severely limited therapy options for these drug-naive patients. Current guidelines for antiretroviral therapy therefore recommend triple combination therapy regimen even for initial treatment. Finally, none of these multidrug therapies completely eliminates HIV and long-term treatment usually results in multidrug resistance.

Therefore there is a continued need for new combinations of active ingredients that are effective against HIV. New types of anti-HIV effective active ingredients, differing in chemical structure and in activity profile are needed to design these new drug combinations. Finding such active ingredients therefore is a highly desirable goal to achieve.

The present invention relates to specific new bisaryl substituted aminopyrimidines. A class of bisaryl substituted pyrimidines has been described in WO-00/27825 as HIV inhibitors. The bisaryl substituted aminopyrimidines of the invention however are structurally different from those known in the prior art and not only act favorably in terms of their capability to inhibit the replication of Human Immunodeficiency Virus (HIV), but also of their improved ability to inhibit the replication of mutant strains, in particular strains which have become resistant to one or more known NNRTI drugs, which strains are referred to as drug or multidrug resistant HIV strains.

The present invention concerns compounds of formula 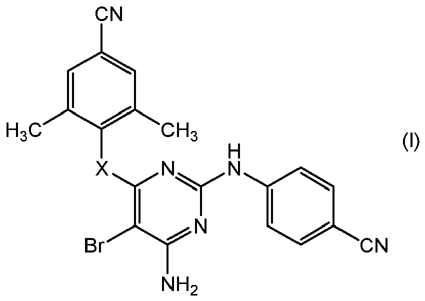 the pharmaceutically acceptable addition salts and stereochemically isomeric forms thereof, wherein
X is NH, S, SO or SO₂.

The present invention also relates to the use of a compound of formula (I) as specified herein for the manufacture of a medicament for the treatment or prevention of HIV infection.

In this specification and claims, a sulfoxide and sulfone may be represented by SO and SO₂ respectively, or by S=O and O=S=O respectively, or by S→O or O←S→O respectively.

For therapeutic use, salts of the compounds of formula (I) are those wherein the counter ion is pharmaceutically acceptable. However, salts of acids and bases, which are non-pharmaceutically acceptable may also find use, for example, in the preparation or purification of a pharmaceutically acceptable compound. All salts, whether pharmaceutically acceptable or not are included within the ambit of the present invention.

The pharmaceutically acceptable addition salts as mentioned hereinabove are meant to comprise the therapeutically active non-toxic acid addition salt forms, which the compounds of formula (I) are able to form. The latter can conveniently be obtained by treating the base form with such appropriate acids as inorganic acids, for example, hydrohalic acids, e.g. hydrochloric, hydrobromic and the like; sulfuric acid; nitric acid; phosphoric acid and the like; or organic acids, for example, acetic, propanoic, hydroxyacetic, 2-hydroxypropanoic, 2-oxopropanoic, oxalic, malonic, succinic, maleic, fumaric, malic, tartaric, 2-hydroxy-1,2,3-propanetricarboxylic, methanesulfonic, ethanesulfonic, benzenesulfonic, 4-methylbenzenesulfonic, cyclohexanesulfamic, 2-hydroxybenzoic, 4-amino-2-hydroxybenzoic and the like acids. Conversely the salt form can be converted by treatment with alkali into the free base form.

Of particular interest are the base-forms of the compounds of formula (I). As used herein, the term "base-form" refers to the compound of formula (I) occurring in its free base form. The term addition salt also comprises the hydrates and solvent addition forms which the compounds of formula (I) are able to form. Examples of such forms are e.g. hydrates, alcoholates and the like.

The term "compounds of formula (I)", or any similar terms such as "compounds of the invention" and the like, is meant to also comprise any quaternary amines of the compounds of formula (I). The term "quaternary amine" defines the quaternary ammonium salts which the compounds of formula (I) are able to form by reaction between a basic nitrogen of a compound of formula (I) and an appropriate quaternizing agent, such as, for example, an optionally substituted alkylhalide, aryl halide or arylalkyl halide, e.g. methyl iodide or benzyl iodide. Other reactants with good leaving groups may also be used, such as alkyl trifluoromethanesulfonates, alkyl methanesulfonates, and alkyl p-toluenesulfonates. A quaternary amine has a positive-charged nitrogen. Pharmaceutically acceptable counterions include chloro, bromo, iodo, trifluoroacetate and acetate. The counterion of choice can be introduced using ion exchange resins.

The term "compounds of formula (I)", or any similar terms such as "compounds of the invention" and the like, is meant to also comprise any N-oxide forms of the compounds of formula (I), which are compounds of formula (I) wherein one or several tertiary nitrogen atoms are oxidized to the N-oxide form.

The terms "stereochemically isomeric forms" or "stereoisomeric forms" as used herein defines all the possible stereoisomeric forms in which the compounds of formula (I) may occur and are intended to be embraced within the scope of this invention. Stereoiosomers may exist where X is SO.

Preferred subgroups of compounds are those compounds of formula (I) as specified above, or any subgroup of compounds of formula (I) specified herein, wherein X is NH or S; or wherein X is NH; or wherein X is S.

The compounds of formula (I) can be prepared by reacting an intermediate of formula (II) or (IV) with an intermediate of formula (III) or (V), as outlined in the following reaction scheme, wherein W represents a suitable leaving group, such as for example halogen, e.g. chloro, bromo and the like.

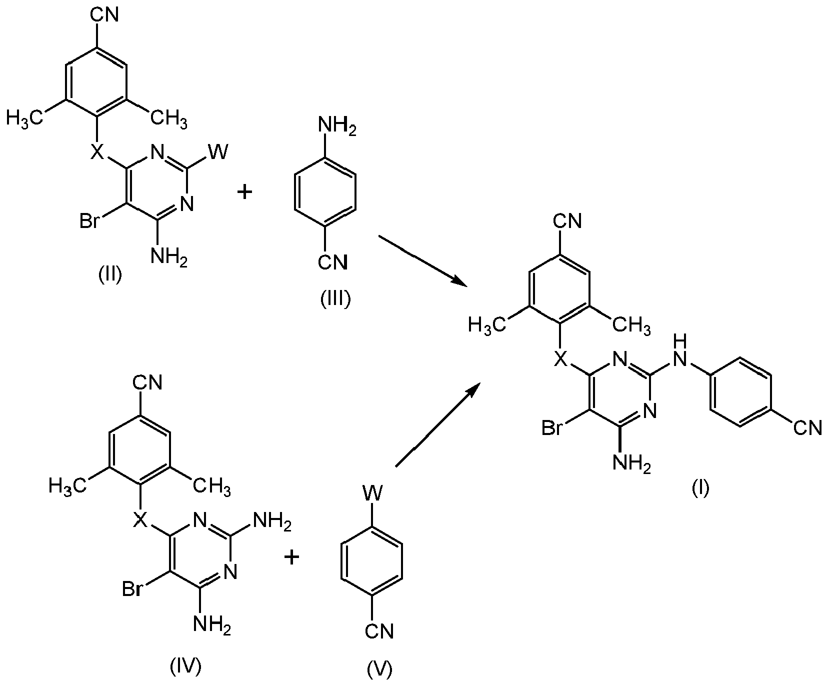

The reaction of the pyrimidine derivative (II), respectively (IV), with the cyanoaniline (III) respectively the cyanophenyl derivative (V), is preferably conducted in a suitable solvent, such as for example an alcohol, e.g. ethanol, 2-propanol; a polar aprotic solvent such as N,N-dimethylformamide; N,N-dimethylacetamide, 1-methyl-2-pyrrolidinone and acetonitrile; an ether such as tetrahydrofuran (THF),1,4-dioxane, propylene glycol monomethylether. These reactions may be done under acid conditions which may be obtained by adding amounts of a suitable acid, e.g. camphor sulfonic acid, and a suitable solvent, such as for example tetrahydrofuran or an alcohol, e.g.ethanol, 1- or 2-propanol, or by using acidified solvents, e.g. hydrochloric acid dissolved in an alkanol such as ethanol, 1- or 2-propanol.

The compounds of formula (I-a), which are compounds of formula (I) wherein X is NR⁴ or S, can also be prepared by reacting a cyanophenyl derivative (VI) with a pyrimidine derivative (VII) or by reacting a cyanophenyl derivative (VIII) with a pyrimidine derivative (IX) as outlined in the following reaction schemes.

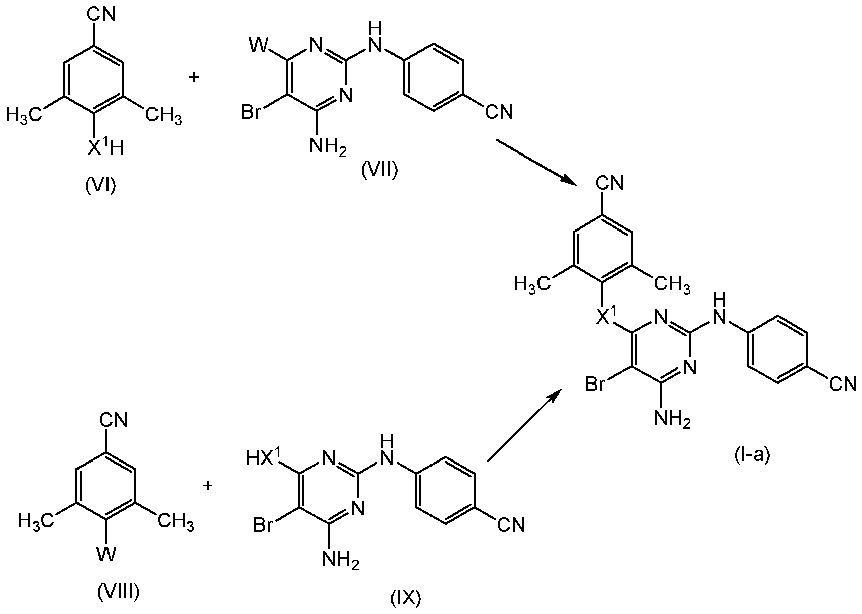

In these reaction schemes X¹ is NR⁴ or S, and W represents a suitable leaving group, such as for example halogen, e.g. chloro and bromo. These reactions preferably are conducted in a suitable solvent, in particular any of the solvents mentioned above in relation to the reaction of (II) with (III).

Still another way to prepare compounds of formula (I) is by brominating a starting material (X) with free bromine, or with a bromine donor such as N-bromo succinimide. This bromination reaction preferably is conducted in a suitable reaction-inert solvent such as an ether, in particular in THF. N-bromo-succinimide can be used in the presence of acetic acid.

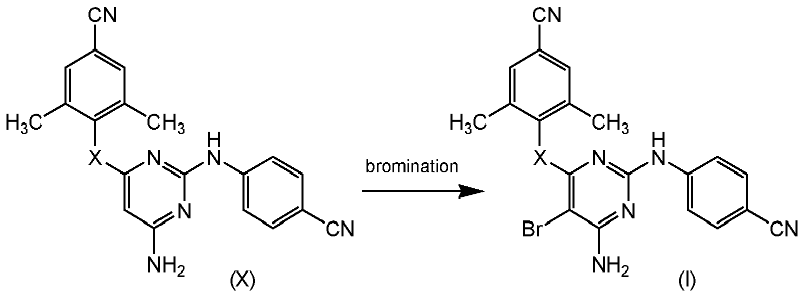

The compounds of formula (I) may further be prepared by converting compounds of formula (I) into each other according to art-known functional group transformation reactions. The compounds of formula (I) wherein X is S can be converted to the corresponding compounds of formula (I) wherein X is SO or SO₂ by an oxidation reaction, in particular by using a suitable peroxide compound. Suitable peroxide compounds comprise hydrogen peroxide or derivatives thereof and peroxy acids. By selecting the oxidant and controlling the reaction circumstances the reaction can be steared towards forming a sulfoxide or a sulfone. If necessary, both can be separated from one another by crystallisation or by column chromatography. Suitable peroxide compounds to obtain sulfoxides are peroxybenzoic acids such as m-chloroperoxybenzoic acid (MCPBA).

The sulfoxide or sulfone moieties can be introduced at an earlier stage of the synthesis, i.e. in one of the intermediates used to prepare the end products of formula (I). In particular, the intermediates of formula (II), (IV) or (X) wherein X is S can be converted to the corresponding intermediates of formula (II), (IV) or (X) wherein X is SO by a controlled oxidation reaction of the starting intermediates with a peroxy acid such as m-chloroperoxybenzoic acid. The thus obtained intermediates (II), (IV) or (X) wherein X is SO are then converted to the corresponding end products (I) wherein X is SO. By using stronger oxidation circumstances, e.g. by using an excess amount of a suitable peroxide, such as the peroxides described hereinafter for the preparation of N-oxides, intermediates wherein X is SO₂ are obtained, which can be converted to the corresponding end products (I) wherein X is SO₂.

The compounds of formula (I) may be converted to the corresponding N-oxide forms following art-known procedures for converting a tertiary nitrogen into its *N*-oxide form. Said N-oxidation reaction may generally be carried out by reacting the starting material of formula (I) with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. tert.butyl hydro-peroxide. Suitable solvents are, for example, water, lower alcohols, e.g. ethanol and the like, hydrocarbons, e.g. toluene, ketones, e.g. 2-butanone, halogenated hydrocarbons, e.g. dichloromethane, and mixtures of such solvents.

The compounds of formula (I) wherein X is SO and some of the precursor intermediates thereof may contain an asymmetric atom. Pure stereochemically isomeric forms of said compounds and said intermediates can be obtained by the application of art-known procedures. Enantiomers can be obtained from racemic mixtures by first converting said racemic mixtures with suitable resolving agents such as, for example, chiral acids, to mixtures of diastereomeric salts or compounds; then physically separating said mixtures of diastereomeric salts or compounds by, for example, selective crystallization or chromatographic techniques, e.g. liquid chromatography and the like methods; and finally converting said separated diastereomeric salts or compounds into the corresponding enantiomers. Pure stereochemically isomeric forms may also be obtained from the pure stereochemically isomeric forms of the appropriate intermediates and starting materials, provided that the intervening reactions occur stereospecifically.

Some of the intermediates and starting materials used to prepare the compounds of formula (I) are known compounds and may be commercially available or may be prepared according to art-known procedures. The synthesis of some of the intermediates is described hereinafter in more detail, wherein in the reaction schemes is as specified for the compounds of formula (I) or any subgroup thereof, X¹ is NR⁴ or S, and W represents a suitable leaving group, in particular chloro or bromo. The bromo group in these intermediates may be replaced by a precursor of a bromo group such as hydroxy or a protected hydroxy (e.g. benzyloxy) which can be converted into a bromo group with a halogenating agent such as POBr₃. This may be done to avoid undesired side reactions.

Intermediates of formula (II-a), which are intermediates of formula (I) wherein X is X¹, can be prepared as outlined in the following reaction scheme. The other intermediates of formula (II) can be derived from intermediates of formula (II-a) wherein X¹ is S by an oxidation reaction to the corresponding sulfoxides or sulfones.

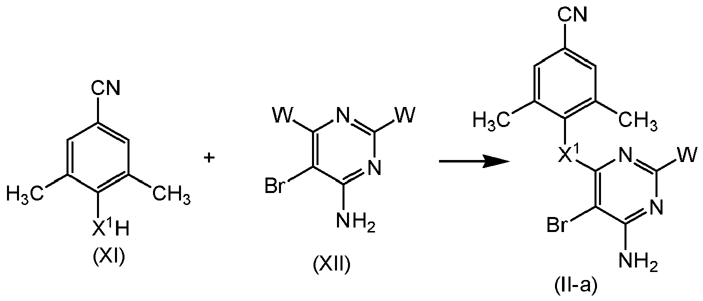

In a similar manner, intermediates (IV-a), which are intermediates of formula (IV) wherein X is X¹, can be prepared starting from a pyrimidine (XIV) as outlined in the following scheme:

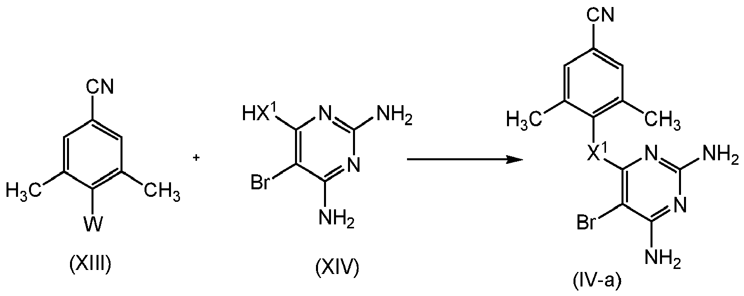

In the above reaction the amino group may or may not be protected by a suitable protective group. The latter comprise benzyl, benzyloxycarbonyl, t-butyloxycarbonyl and the like.

Intermediates (IX) can be prepared by condensing a pyrimidine derivative (XV) with 4-aminobenzonitrile as outlined in the following scheme. If desired to avoid side reactions, the -X¹H and/or amino group may be protected and the bromine may be replaced by a precursor of bromine as set forth above, the resulting intermediates (IX') encompassing intermediates (IX) or precursors of intermediates (IX).

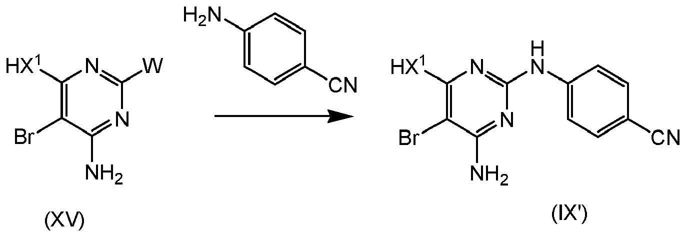

Intermediates of formula (X) can be prepared as outlined in the following reaction scheme.

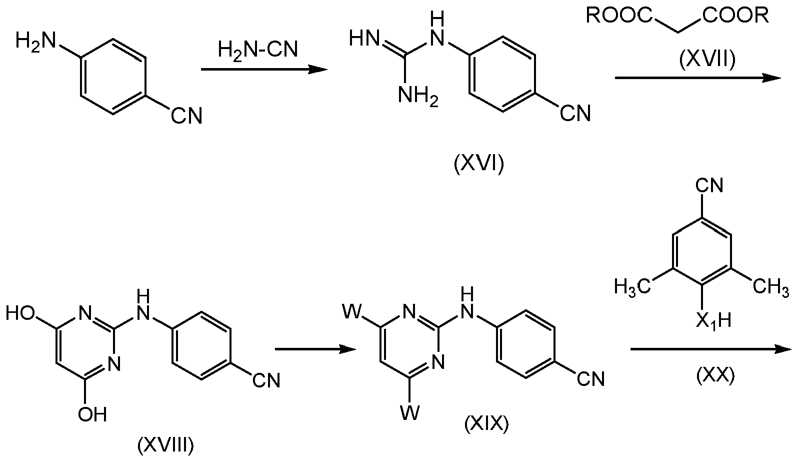

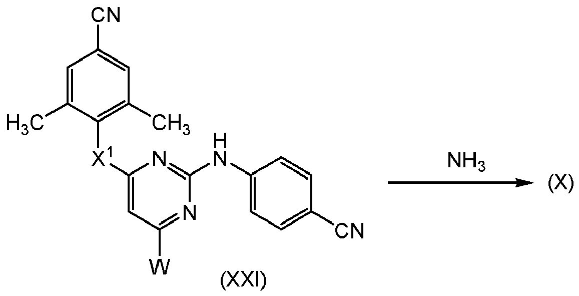

First 4-aminobenzonitrile is reacted with cyanamid to yield 4-cyanophenyl guanidine (XVI). This reaction may be conducted in water in the presence of a strong acid, e.g. hydrochloric acid, at increased temperature, e.g. at about 50°C to 70°C, e.g. at about 60°C. The latter is reacted with a diC₁₋₆alkyl malonic ester of formula (XVII), each R independently being C₁₋₆alkyl, preferably each R is methyl. This reaction may be conducted in a suitable solvent, e.g. an alcohol such as methanol, in the presence of a strong base such as an alkali metal alkoxide, e.g. sodium methoxide, at increased temperature such as at reflux temperature.

The thus obtained 4,6-dihydroxypyrimidine (XVIII) is converted to the pyrimidine derivative (XIX) wherein each W is a leaving group and in particular is halo, preferably chloro or bromo. This conversion can be done by using a suitable halogenating agent such as POCl₃ or POBr₃ This reaction may be conducted in a suitable solvent, in particular a polar aprotic solvent, e.g. in DMF, DMA, HMPT, N-methylpyrrolidone, DMSO and the like, preferably in acetonitrile. The reaction may be conducted at increased temperature, preferably at reflux temperature. Other leaving groups can be introduced following art-know alcohol to leaving group conversion reactions.

The pyrimidine derivative (XIX) is reacted with a 4-substituted benzonitrile (XX) wherein X¹ is as specified above to yield the desired intermediates (XXI). The reaction of (XIX) with (XX) may be conducted in a suitable solvent such as an ether, e.g THF, a halogenated hydrocarbon, e.g. CH₂Cl₂, CHCl₃ and in particular a polar aprotic solvent, e.g. in DMF, DMA, HMPT, acetonitrile, DMSO and the like, and preferably in N-methylpyrrolidone. A base may be added to pick up the acid that is liberated during the course of the reaction, e.g. an alkali metal carbonate such as potassium carbonate. The reaction of (XIX) with (XX) may be conducted ad a slightly increased temperature, for example at about 30°C to 50°C, e.g. at about 40°C.

The intermediates of formula (X), or the acid-addition salts thereof, can be prepared by reacting an intermediate (XXI) as specified above with an amino group introducing agent. This reaction preferably is conducted in a suitable solvent such as an alcohol, e.g. methanol or ethanol, an ether, e.g. THF or a ethylene or propylene glycol ether such as ethylene glycol monomethyl ether, propylene glycol monomethyl ether (PGMME), a polar aprotic solvent, e.g. DMF, DMA, HMPT, acetonitrile, DMSO and the like, and in particular in N-methylpyrrolidone. Suitable amino group introducing agents comprise ammonia in liquid or gaseous form, ammonia dissolved in water or dissolved in an organic solvent such as an alcohol, e.g. methanol or ethanol, or in a polar aprotic solvent such as in DMF, DMA, HMPT, acetonitrile, DMSO and the like. Alternatively the amine group introducing agent may be a benzylamine, whereby the reaction yields a benzylamino group. The latter can be converted to an amino group by a suitable de-benzylation reaction, e.g. by the benzylamino compound to a catalytic hydrogenation reaction, e.g. hydrogen in the presence of a noble metal catalyst such as Pd.

A preferred amino group introducing agent is ammonia. It is added to the mixture of the starting material and the solvent preferably at increased temperature, for example at a temperature of 100°C to the boiling point of the reaction mixture, in particular between 120°C and 160°C, e.g. at about 140°C to 150°C, and at higher pressure, e.g. at a increased pressure such as a pressure between about 2 and 10 Bar, in particular between about 3 and 8 Bar, e.g. at about 4 - 5 Bar. The reaction mixture may be kept at this temperature for several hours, for example a period between about 5 and 24 hours, or between about 8 - 10 hours. In some instances where the reactivity of the W group is lower, the reaction mixture is kept at this temperature for one or several days, e.g. between one and 10 days, in particular between 2 and 8 days.

The reaction may be terminated by pouring the reaction mixture in hot water, e.g. at about 100°C. The desired product is allowed to crystallize, for example by adding a further amount of water and allowing the mixture to cool. If available, the mixture may be seeded with a small amount of the end product, which may have been obtained from a previous reaction procedure, in order to facilitate the crystallization process.

The intermediates of formula (X) wherein X is SO or SO₂ can be prepared from the corresponding intermediates (X) wherein X is S by a suitable oxidation reaction, as described above.

The intermediates of formula (XX-a), which are intermediates of formula (XX) wherein -X¹H is -SH can be prepared from the corresponding 4-hydroxybenzonitriles (XXV) as outlined in the following reaction scheme.

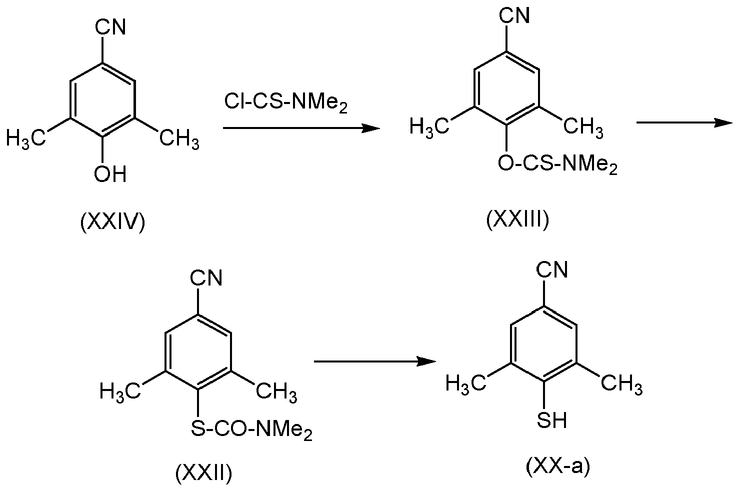

Intermediates (XXIV) are first reacted with dimethylthiocarbamoyl chloride to yield intermediates (XXIII), which are converted to intermediates (XXII) by a thermic conversion by heating the intermediates (XXIII). Intermediates (XXII) are reacted with a base, in particular NaOH or KOH, to yield the mercapto derivatives (XX-a).

The compounds of formula (I) show antiretroviral properties (reverse transcriptase inhibiting properties), in particular against Human Immunodeficiency Virus (HIV), which is the aetiological agent of Acquired Immune Deficiency Syndrome (AIDS) in humans. The HIV virus preferentially infects human T-4 cells and destroys them or changes their normal function, particularly the coordination of the immune system. As a result, an infected patient has an ever decreasing number of T-4 cells, which moreover behave abnormally. Hence, the immunological defense system is unable to combat infections and neoplasms and the HIV infected subject usually dies by opportunistic infections such as pneumonia, or by cancers. Other conditions associated with HIV infection include thrombocytopaenia, Kaposi's sarcoma and infection of the central nervous system characterized by progressive demyelination, resulting in dementia and symptoms such as, progressive dysarthria, ataxia and disorientation. HIV infection further has also been associated with peripheral neuropathy, progressive generalized lymphadenopathy (PGL) and AIDS-related complex (ARC).

The present compounds also show activity against (multi) drug resistant HIV strains, in particular (multi) drug resistant HIV-1 strains, more in particular the present compounds show activity against HIV strains, especially HIV-1 strains, that have acquired resistance to one or more art-known non-nucleoside reverse transcriptase inhibitors. Art-known non-nucleoside reverse transcriptase inhibitors are those non-nucleoside reverse transcriptase inhibitors other than the present compounds and known to the person skilled in the art, in particular commercial non-nucleoside reverse transcriptase inhibitors. The present compounds also have little or no binding affinity to human α,-1 acid glycoprotein; human α,-1 acid glycoprotein does not or only weakly affect the anti HIV activity of the present compounds.

Due to their antiretroviral properties, particularly their anti-HIV properties, especially their anti-HIV-1-activity, the compounds of formula (I) are useful in the treatment of individuals infected by HIV and in the prophylaxis of HIV infection. In general, the compounds of the present invention may be useful in the treatment of warm-blooded animals infected with viruses whose existence is mediated by, or depends upon, the enzyme reverse transcriptase. Conditions which may be prevented or treated with the compounds of the present invention, especially conditions associated with HIV and other pathogenic retroviruses, include AIDS, AIDS-related complex (ARC), progressive generalized lymphadenopathy (PGL), as well as chronic Central Nervous System diseases caused by retroviruses, such as, for example HIV mediated dementia and multiple sclerosis.

The compounds of the present invention or any subgroup thereof may therefore be used as medicines against above-mentioned conditions. Said use as a medicine or method of treatment comprises the administration to HIV-infected subjects of an amount effective to combat the conditions associated with HIV and other pathogenic retroviruses, especially HIV-1. In particular, the compounds of formula (I) may be used in the manufacture of a medicament for the treatment or the prevention of HIV infections.

In view of the utility of the compounds of formula (I), there is provided a method of treating warm-blooded animals, including humans, suffering from or a method of preventing warm-blooded animals, including humans, to suffer from viral infections, especially HIV infections. Said method comprises the administration, preferably oral administration, of an effective amount of a compound of formula (I), a N-oxide form, a pharmaceutically acceptable addition salt, a quaternary amine or a possible stereoisomeric form thereof, to warm-blooded animals, including humans.

The present invention also provides compositions for treating viral infections comprising a therapeutically effective amount of a compound of formula (I) and a pharmaceutically acceptable carrier or diluent.

The compounds of the present invention or any subgroup thereof may be formulated into various pharmaceutical forms for administration purposes. As appropriate compositions there may be cited all compositions usually employed for systemically administering drugs. To prepare the pharmaceutical compositions of this invention, an effective amount of the particular compound, optionally in addition salt form, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, particularly, for administration orally, rectally, percutaneously, or by parenteral injection. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, diluents, lubricants, binders, disintegrating agents and the like in the case of powders, pills, capsules, and tablets. Because of their ease in administration, tablets and capsules represent the most advantageous oral dosage unit forms, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations, which are intended to be converted, shortly before use, to liquid form preparations. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin. Said additives may facilitate the administration to the skin and/or may be helpful for preparing the desired compositions. These compositions may be administered in various ways, e.g., as a transdermal patch, as a spot-on, as an ointment. The compounds of the present invention may also be administered via inhalation or insufflation by means of methods and formulations employed in the art for administration via this way. Thus, in general the compounds of the present invention may be administered to the lungs in the form of a solution, a suspension or a dry powder. Any system developed for the delivery of solutions, suspensions or dry powders via oral or nasal inhalation or insufflation are suitable for the administration of the present compounds.

To aid solubility of the compounds of formula (I), suitable ingredients, e.g. cyclodextrins, may be included in the compositions. Appropriate cyclodextrins are α-, β-, γ-cyclodextrins or ethers and mixed ethers thereof wherein one or more of the hydroxy groups of the anhydroglucose units of the cyclodextrin are substituted with C₁₋₆alkyl, particularly methyl, ethyl or isopropyl, e.g. randomly methylated β-CD; hydroxy-C₁₋₆alkyl, particularly hydroxyethyl, hydroxy-propyl or hydroxybutyl; carboxy-C₁₋₆alkyl, particularly carboxymethyl or carboxyethyl; C₁₋₆alkylcarbonyl, particularly acetyl. Especially noteworthy as complexants and/or solubilizers are β-CD, randomly methylated β-CD, 2,6-dimethyl-β-CD, 2-hydroxyethyl-β-CD, 2-hydroxyethyl-β-CD, 2-hydroxypropyl-β-CD and (2-carboxymethoxy)propyl-β-CD, and in particular 2-hydroxypropyl-β-CD (2-HP-β-CD).

The term mixed ether denotes cyclodextrin derivatives wherein at least two cyclodextrin hydroxy groups are etherified with different groups such as, for example, hydroxy-propyl and hydroxyethyl.

The average molar substitution (M.S.) is used as a measure of the average number of moles of alkoxy units per mole of anhydroglucose. The average substitution degree (D.S.) refers to the average number of substituted hydroxyls per anhydroglucose unit. The M.S. and D.S. value can be determined by various analytical techniques such as nuclear magnetic resonance (NMR), mass spectrometry (MS) and infrared spectroscopy (IR). Depending on the technique used, slightly different values may be obtained for one given cyclodextrin derivative. Preferably, as measured by mass spectrometry, the M.S. ranges from 0.125 to 10 and the D.S. ranges from 0.125 to 3.

Other suitable compositions for oral or rectal administration comprise particles consisting of a solid dispersion comprising a compound of formula (I) and one or more appropriate pharmaceutically acceptable water-soluble polymers.

The term "a solid dispersion" used hereinafter defines a system in a solid state (as opposed to a liquid or gaseous state) comprising at least two components, in casu the compound of formula (I) and the water-soluble polymer, wherein one component is dispersed more or less evenly throughout the other component or components ( in case additional pharmaceutically acceptable formulating agents, generally known in the art, are included, such as plasticizers, preservatives and the like). When said dispersion of the components is such that the system is chemically and physically uniform or homogenous throughout or consists of one phase as defined in thermo-dynamics, such a solid dispersion will be called "a solid solution". Solid solutions are preferred physical systems because the components therein are usually readily bioavailable to the organisms to which they are administered. This advantage can probably be explained by the ease with which said solid solutions can form liquid solutions when contacted with a liquid medium such as the gastro-intestinal juices. The ease of dissolution may be attributed at least in part to the fact that the energy required for dissolution of the components from a solid solution is less than that required for the dissolution of components from a crystalline or microcrystalline solid phase.

The term "a solid dispersion" also comprises dispersions, which are less homogenous throughout than solid solutions. Such dispersions are not chemically and physically uniform throughout or comprise more than one phase. For example, the term "a solid dispersion" also relates to a system having domains or small regions wherein amorphous, microcrystalline or crystalline compound of formula (I), or amorphous, microcrystalline or crystalline water-soluble polymer, or both, are dispersed more or less evenly in another phase comprising water-soluble polymer, or compound of formula (I), or a solid solution comprising compound of formula (I) and water-soluble polymer. Said domains are regions within the solid dispersion distinctively marked by some physical feature, small in size, and evenly and randomly distributed throughout the solid dispersion.

Various techniques exist for preparing solid dispersions including melt-extrusion, spray-drying and solution-evaporation. After preparing the solid dispersions, the obtained products can be optionally milled and sieved. The solid dispersion product may be milled or ground to particles having a particle size of less than 600 µm, preferably less than 400 µm and most preferably less than 125 µm.

The particles prepared as described hereinabove can then be formulated by conventional techniques into pharmaceutical dosage forms such as tablets and capsules.

The water-soluble polymers in the particles are polymers that have an apparent viscosity, when dissolved at 20°C in an aqueous solution at 2 % (w/v), of 1 to 5000 mPa.s more preferably of 1 to 700 mPa.s, and most preferred of 1 to 100 mPa.s. For example, suitable water-soluble polymers include alkylcelluloses, hydroxyalkylcelluloses, hydroxyalkyl alkylcelluloses, carboxyalkylcelluloses, alkali metal salts of carboxyalkylcelluloses, carboxyalkylalkylcelluloses, carboxyalkylcellulose esters, starches, pectines, chitin derivates, di-, oligo- and polysaccharides such as trehalose, alginic acid or alkali metal and ammonium salts thereof, carrageenans, galactomannans, tragacanth, agar-agar, gum arabic, guar gum and xanthan gum, polyacrylic acids and the salts thereof, polymethacrylic acids and the salts thereof, methacrylate copolymers, polyvinylalcohol, polyvinylpyrrolidone, copolymers of polyvinylpyrrolidone with vinyl acetate, combinations of polyvinylalcohol and polyvinylpyrrolidone, polyalkylene oxides and copolymers of ethylene oxide and propylene oxide. Preferred water-soluble polymers are hydroxypropyl methylcelluloses.

Also one or more cyclodextrins can be used as water-soluble polymer in the preparation of the above-mentioned particles as is disclosed in WO 97/18839. These cyclodextrins include the pharmaceutically acceptable unsubstituted and substituted cyclodextrins known in the art, more particularly α, β or γ cyclodextrins or the pharmaceutically acceptable derivatives thereof.

Substituted cyclodextrins that can be used to prepare the particles described above include polyethers described in U.S. Patent 3,459,731. Further substituted cyclodextrins are ethers wherein the hydrogen of one or more cyclodextrin hydroxy groups is replaced by C₁₋₆alkyl, hydroxy C₁₋₆alkyl, carboxyC₁₋₆alkyl or C₁₋₆alkyloxycarbonyl C₁₋₆alkyl or mixed ethers thereof. In particular such substituted cyclodextrins are ethers wherein the hydrogen of one or more cyclodextrin hydroxy groups is replaced by C₁₋₃alkyl, hydroxyC₂₋₄alkyl or carboxy C₁₋₂alkyl or more in particular by methyl, ethyl, hydroxyethyl, hydroxypropyl, hydroxybutyl, carboxy-methyl or carboxyethyl.

Of particular utility are the β-cyclodextrin ethers, e.g. dimethyl-β-cyclodextrin, and polyethers, e.g. hydroxypropyl β-cyclodextrin and hydroxyethyl β-cyclodextrin. Such an alkyl ether may be a methyl ether with a degree of substitution of about 0.125 to 3, e.g. about 0.3 to 2. Such a hydroxypropyl cyclodextrin may for example be formed from the reaction between β-cyclodextrin an propylene oxide and may have a MS value of about 0.125 to 10, e.g. about 0.3 to 3.

Another class of substituted cyclodextrins that can be used are the sulfobutylcyclodextrines.

The ratio of the compound of formula (I) to the water-soluble polymer may vary widely. For example ratios of 1/100 to 100/1 may be applied. Interesting ratios of a compound of formula (I) to cyclodextrin range from about 1/10 to 10/1. More interesting ratios range from about 1/5 to 5/1.

It may further be convenient to formulate the compounds of formula (I) in the form of nanoparticles which have a surface modifier adsorbed on the surface thereof in an amount sufficient to maintain an effective average particle size of less than 1000 nm. Useful surface modifiers are believed to include those which physically adhere to the surface of the compound of formula (I) but do not chemically bond to said compound. Suitable surface modifiers may be selected from known organic and inorganic pharmaceutical excipients. Such excipients include various polymers, low molecular weight oligomers, natural products and surfactants. Preferred surface modifiers include nonionic and anionic surfactants.

Yet another interesting way of formulating the compounds of formula (I) involves incorporating compounds of formula (I) in hydrophilic polymers and applying this mixture as a coat film on small beads, which can be further processed into a composition for oral administration. These beads comprise a central, rounded or spherical core, a coating film of a hydrophilic polymer and a compound of formula (I) and optionally a seal-coating layer. Materials suitable for use as cores in these beads are manifold, provided that said materials are pharmaceutically acceptable and have appropriate dimensions and firmness. Examples of such materials are polymers, inorganic substances, organic substances, and saccharides and derivatives thereof.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, powder packets, wafers, suppositories, injectable solutions or suspensions and the like, and segregated multiples thereof.

Those of skill in the treatment of HIV-infection could determine the effective daily amount from the test results presented here. In general it is contemplated that an effective daily amount would be from 0.01 mg/kg to 50 mg/kg body weight, more preferably from 0.1 mg/kg to 10 mg/kg body weight. It may be appropriate to administer the required dose as two, three, four or more sub-doses at appropriate intervals throughout the day. Said sub-doses may be formulated as unit dosage forms, for example, containing 1 to 1000 mg, and in particular 5 to 200 mg of active ingredient per unit dosage form.

The exact dosage and frequency of administration depends on the particular compound of formula (I) used, the particular condition being treated, the severity of the condition being treated, the age, weight and general physical condition of the particular patient as well as other medication the individual may be taking, as is well known to those skilled in the art. Furthermore, it is evident that said effective daily amount may be lowered or increased depending on the response of the treated subj ect and/or depending on the evaluation of the physician prescribing the compounds of the instant invention. The effective daily amount ranges mentioned hereinabove are therefore only guidelines and are not intended to limit the scope or use of the invention to any extent.

The present compounds of formula (I) can be used alone or in combination with other therapeutic agents, such as anti-virals, antibiotics, immunomodulators or vaccines for the treatment of viral infections. They may also be used alone or in combination with other prophylactic agents for the prevention of viral infections. The present compounds may be used in vaccines and methods for protecting individuals against viral infections over an extended period of time. The compounds may be employed in such vaccines either alone or together with other compounds of this invention or together with other anti-viral agents in a manner consistent with the conventional utilization of reverse transcriptase inhibitors in vaccines. Thus, the present compounds may be combined with pharmaceutically acceptable adjuvants conventionally employed in vaccines and administered in prophylactically effective amounts to protect individuals over an extended period of time against HIV infection.

Also, the combination of one or more additional antiretroviral compounds and a compound of formula (I) can be used as a medicine. Thus, the present invention also relates to a product containing (a) a compound of formula (I), and (b) one or more additional antiretroviral compounds, as a combined preparation for simultaneous, separate or sequential use in anti-HIV treatment. The different drugs may be combined in a single preparation together with pharmaceutically acceptable carriers. Said other antiretroviral compounds may be any known antiretroviral compounds such as suramine, pentamidine, thymopentin, castanospermine, dextran (dextran sulfate), foscarnet-sodium (trisodium phosphono formate); nucleoside reverse transcriptase inhibitors (NRTIs), e.g. zidovudine (AZT), didanosine (ddI), zalcitabine (ddC), lamivudine (3TC), stavudine (d4T), emtricitabine (FTC), abacavir (ABC), D-D4FC (Reverset^{™}), alovudine (MIV-310), amdoxovir (DAPD), elvucitabine (ACH-126,443), and the like; non-nucleoside reverse transcriptase inhibitors (NNRTIs) such as delarvidine (DLV), efavirenz (EFV), nevirapine (NVP), capravirine (CPV), calanolide A, TMC120, etravirine (TMC125), TMC278, BMS-561390, DPC-083 and the like; nucleotide reverse transcriptase inhibitors (NtRTIs), e.g. tenofovir (TDF) and tenofovir disoproxil fumarate, and the like; compounds of the TIBO (tetrahydroimidazo-[4,5,1-jk][1,4]-benzodiazepine-2(1*H*)-one and thione)-type e.g. (S)-8-chloro-4,5,6,7-tetrahydro-5-methyl-6-(3-methyl-2-butenyl)imidazo-[4,5,1-jk][1,4]benzodiazepine-2(1*H*)-thione; compounds of the α,-APA (α,-anilino phenyl acetamide) type e.g. α,-[(2-nitrophenyl)amino]-2,6-dichlorobenzene-acetamide and the like; inhibitors of trans-activating proteins, such as TAT-inhibitors, e.g. RO-5-3335; REV inhibitors; protease inhibitors e.g. ritonavir (RTV), saquinavir (SQV), lopinavir (ABT-378 or LPV), indinavir (IDV), amprenavir (VX-478), TMC-126, BMS-232632, VX-175, DMP-323, DMP-450 (Mozenavir), nelfinavir (AG-1343), atazanavir (BMS 232,632), palinavir, TMC-114, RO033-4649, fosamprenavir (GW433908 or VX-175), P-1946, BMS 186,318, SC-55389a, L-756,423, tipranavir (PNU-140690), BILA 1096 BS, U-140690, and the like; entry inhibitors which comprise fusion inhibitors (e.g. T-20, T-1249), attachment inhibitors and co-receptor inhibitors; the latter comprise the CCR5 antagonists and CXR4 antagonists (e.g. AMD-3100); examples of entry inhibitors are enfuvirtide (ENF), GSK-873,140, PRO-542, SCH-417,690, TNX-355, maraviroc (UK-427,857); a maturation inhibitor for example is PA-457 (Panacos Pharmaceuticals); inhibitors of the viral integrase; ribonucleotide reductase inhibitors (cellular inhibitors), e.g. hydroxyurea and the like.

By administering the compounds of the present invention with other anti-viral agents, which target different events in the viral life cycle, the therapeutic effect of these compounds can be potentiated. Combination therapies as described above exert a synergistic effect in inhibiting HIV replication because each component of the combination acts on a different site of HIV replication. The use of such combinations may reduce the dosage of a given conventional anti-retroviral agent which would be required for a desired therapeutic or prophylactic effect as compared to when that agent is administered as a monotherapy. These combinations may reduce or eliminate the side effects of conventional single anti-retroviral therapy while not interfering with the anti-viral activity of the agents. These combinations reduce potential of resistance to single agent therapies, while minimizing any associated toxicity. These combinations may also increase the efficacy of the conventional agent without increasing the associated toxicity.

The compounds of the present invention may also be administered in combination with immunomodulating agents, e.g. levamisole, bropirimine, anti-human alpha interferon antibody, interferon alpha, interleukin 2, methionine enkephalin, diethyldithiocarbamate, tumor necrosis factor, naltrexone and the like; antibiotics, e.g. pentamidine isethiorate and the like; cholinergic agents, e.g. tacrine, rivastigmine, donepezil, galantamine and the like; NMDA channel blockers, e.g. memantine to prevent or combat infection and diseases or symptoms of diseases associated with HIV infections, such as AIDS and ARC, e.g. dementia. A compound of formula (I) can also be combined with another compound of formula (I).

Although the present invention focuses on the use of the present compounds for preventing or treating HIV infections, the present compounds may also be used as inhibitory agents for other viruses, which depend on similar reverse transcriptases for obligatory events in their life cycle.

The following examples are intended to illustrate the present invention and not to limit its scope thereto.

### Examples

### Example 1: preparation of 4-amino-3,5-dimethylbenzonitrile

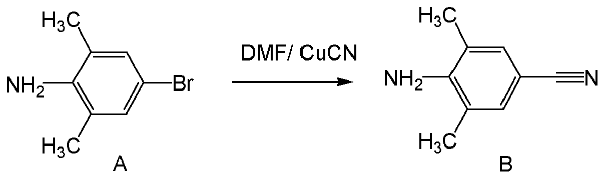

75g of intermediate A, 180ml of DMF and 40.2g CuCN were mixed and heated for 4 hours at 150°C. Subsequently the whole was cooled down to 90°C and 500ml of 1,2-diaminopropane was added and the mixture was stirred for 30 min after which it was cooled down to 20°C. The precipitate that was formed was removed by filtration and the product that was filtered off was dissolved in 3.01 of dichloromethane. 200g silicagel was added to this solution and the whole was stirred for 30min. The silicagel was removed by filtration and the solvent was evaporated at 50°C, yielding 43g of 4-amino-3,5-dimethylbenzonitrile (intermediate B).
Quality: Lc area%: >95%.

### Example 2

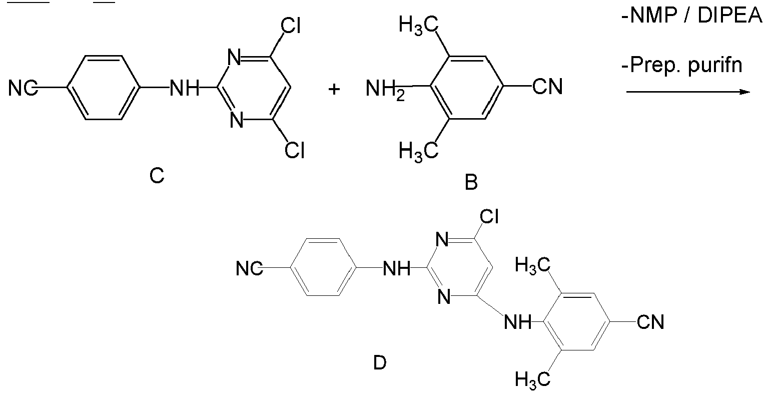

77.98g of intermediate C, which is a known product, 43g of intermediate B, 300 ml of NMP and 43.7g diisopropyl ethylamine were mixed and stirred for 48h at 155°C. The mixture was cooled down to 90°C and 90 ml water was added. The end product was allowed crystallize by slowly cooling down to 25°C. Subsequently the end product was isolated by filtration and dried at 50°C. The thus isolated product was purified by column chromatography over silicagel. Yield: 2.8g of intermediate D.
Quality: Lc area%: >98%

### Example 3

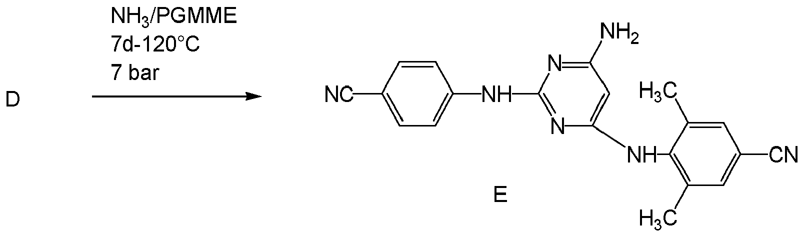

2.16g of intermediate D and 200 ml of propylene glycol monomethyl ether (PGMME) were mixed and subsequently NH₃ gas was added at 1 bar. The mixture was heated to 145°C and further NH₃ was added until a pressure of 4.5 bar was reached. The whole was stirred for 20h at 145°C while keeping under 4.5 bar NH₃ atmosphere. Subsequently the mixture was stirred additionally for 6 days at 120°C and 7 bar of NH₃ atmosphere. Thereafter the mixture was cooled to 20°C and the solvent was removed by evaporation, yielding : 3.2g of intermediate E.
Quality: Lc area%: 95.3%

### Example 4: preparation of compound 1

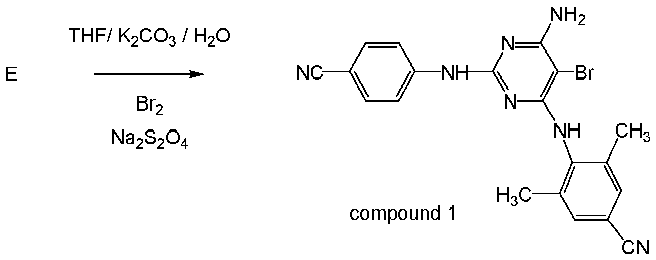

3.2g of intermediate E was mixed with 150 ml of THF, 50 ml of water and 1.6g of K₂CO₃ at 25°C. After cooling to 0°C, 1.03g of Br₂ was added to the mixture during 10 min while keeping the temperature at 0°C . Subsequently, the excess Br₂ was decomposed with aqueous Na₂S₂O₄. The water layer was removed and the organic layer was evaporated. The residue was purified by column chromatography, yielding 1.3g of compound 1.
Quality: Lcwt%: 98.8%.

### Example 5

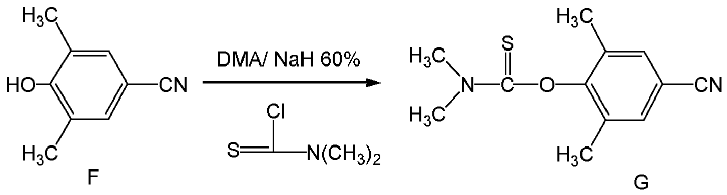

A mixture of 21.91g of intermediate F, 200 ml DMA and 14.9g NaH 60% was stirred for 1 hour at 70°C. After cooling down to 25°C, the mixture was poured out in 2 L of water. The pH of the thus obtained aqueous mixture was brought to pH 2 by adding hydrochloric acid. The end product G was isolated by filtration, yielding 36.92g of compound G.
Quality: GC area%: 100%

### Example 6

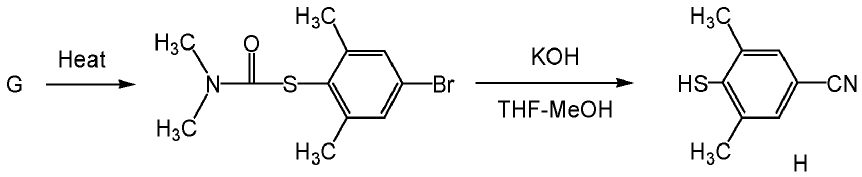

36.92g of intermediate G was heated to 220-240°C for 1hour. After cooling to room temperature, 50ml of THF, 50ml of MeOH and 16.9g of KOH were added to the mixture. After stirring for 1h at 50°C, the reaction mixture was decomposed with 2L of water. The pH of the thus obtained aqueous mixture was brought to pH 2 by adding hydrochloric acid. The end product was filtered off, yielding 13.25g of compound H. Quality: Gc: 100% P

### Example 7

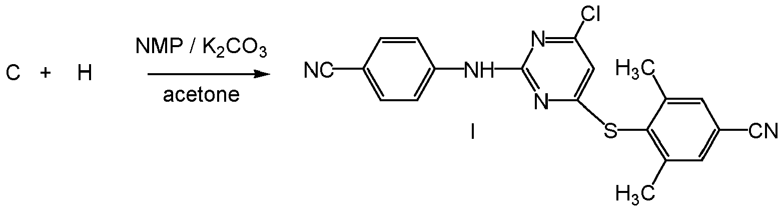

A mixture of 17.23g of intermediate C, 11.61g of intermediate H, 52 ml of NMP and 9.9g of K₂CO₃ was mixed and stirred for 45min at 40°C. After the addition of 44 ml of water, the desired end product was allowed to crystallize by slowly cooling down to 25°C. The end product was isolated by filtration and dried at 50°C. The wet product was reslurried in 65ml acetone and the product was isolated by filtration and drying at 50°C, yielding 19.43g of intermediate I.
Quality: Lc area%: 77% product

### Example 8

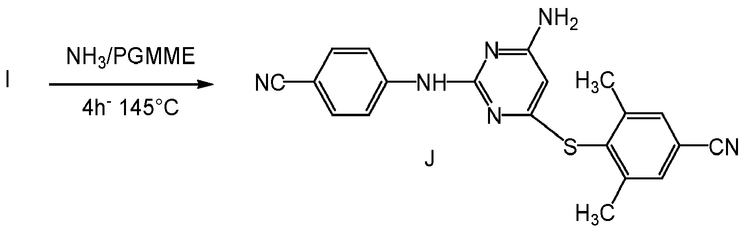

A mixture of 3g of intermediate I as prepared in the previous example and 30 ml NMP was heated to 145°C. NH₃ gas was added until a pressure of 4.5 bar was reached. After stirring for 4h at 145°C while keeping the pressure at 4.5 bar NH₃ atmosphere, the solvent was removed by evaporation, yielding 1.8g of intermediate J.
Quality: Lc area%: 81%

### Example 9

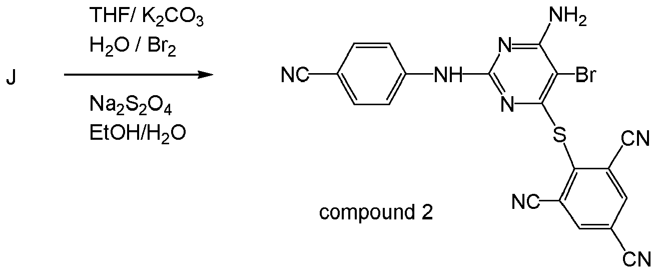

A mixture of 1.46g of intermediate J was mixed with 150ml CH₂Cl₂, 50 ml water and 0.68 g of K₂CO₃ at 25°C. After cooling to 0°C, 0.93g of Br₂ was added at 0°C during 10 minutes. The thus obtained mixture was stirred for 1h at 0°C. The excess Br₂ was decomposed with aqueous Na₂S₂O₄. The water layer was removed and the organic layer was evaprorated. The residue was purified by column chromatography, yielding 1g of compound 2.
Quality: Lc area%: 98%

### Example 10

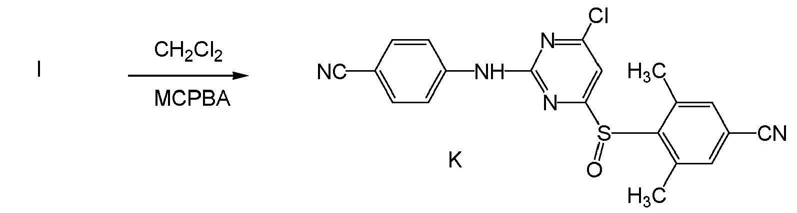

A mixture of 9g of intermediate I, 500 ml of CH₂Cl₂ and 7.28g of m-chloroperoxybenzoic acid (MCPBA) was stirred for 16h at reflux temperatuare. The mixture was subsequently washed with NaOH at room temperature and the solvent was evaporated, yielding 11.45g of intermediate K.
Quality: Lc area%: 62.3% intermediate K
and 24.9% of the corresponding SO₂-product

### Example 11

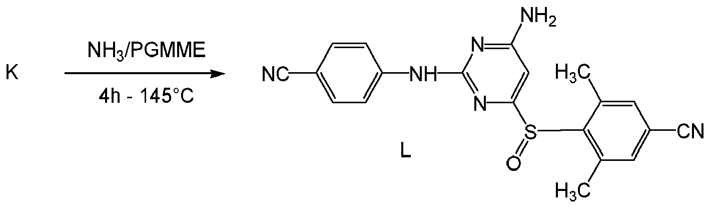

A mixture of 11g of intermediate K (as obtained in the previous example) and 300 ml THF was heated to 40°C and NH₃ gas was added until a pressure of 4.5 bar. After stirring for 2 hours at 40°C and 4.5 bar NH₃ atmosphere, 50 ml of NMP was added and the whole was stirred for an additional 4h at 40°C and 4.5 bar NH₃ atmosphere. After completion of the reaction, the solvent was evaporated. The product was extracted from the residue with 2.5 l of CH₂Cl₂ and washed with 21 of water. After separation, the solvent of the organic layer was evaporated, yielding 6g of product L.
Quality: Lc area%: 34% P

### Example 12

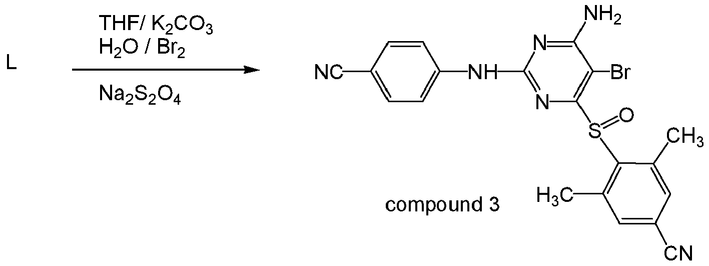

3.5g of intermediate L (as obtained in the previous example), 150 ml of THF, 10 ml of water and 1.1g of K₂CO₃ were mixed at 25°C. After cooling to 0°C, 4.04g of Br₂ was added at 0°C during 10 min. The mixture was stirred for 1h at 0°C and subsequently the excess Br₂ was decomposed with aqua Na₂S₂O₄. The separated organic layer was evaporated and the residue was purified by column chromatography, yielding 0.58g of compound 3.
Quality: Lc area%: 98%

### Example 13 Formulations

### Capsules

A compound 1 is dissolved in organic solvent such as ethanol, methanol or methylene chloride, preferably, in a mixture of ethanol and methylene chloride. Polymers such as polyvinylpyrrolidone copolymer with vinyl acetate (PVP-VA) or hydroxypropylmethylcellulose (HPMC), typically 5 mPa.s, are dissolved in organic solvents such as ethanol, methanol methylene chloride. Preferably the polymer is dissolved in ethanol. The polymer and compound solutions are mixed and subsequently spray dried. The ratio of compound/polymer is selected from 1/1 to 1/6. Intermediate ranges can be 1/1.5 and 1/3. A suitable ratio can be 1/6. The spray-dried powder, a solid dispersion, is subsequently filled in capsules for administration. The drug load in one capsule ranges between 50 and 100 mg depending on the capsule size used.

### Film-coated Tablets

### Preparation of Tablet Core

A mixture of 1000 g of a compound 1, 2850 g lactose and 1000 g starch are mixed well and thereafter humidified with a solution of 25 g sodium dodecyl sulfate and 50 g polyvinylpyrrolidone in about 1000 ml of water. The wet powder mixture is sieved, dried and sieved again. Then there is added 500 g microcrystalline cellulose and 75 g hydrogenated vegetable oil. The whole is mixed well and compressed into tablets, giving 10,000 tablets, each comprising 100 mg of the active ingredient.

### Coating

To a solution of 10 g methylcellulose in 75 ml of denaturated ethanol there is added a solution of 5 g of ethylcellulose in 150 ml of dichloromethane. Then there is added 75 ml of dichloromethane and 2.5 ml 1,2,3-propanetriol. 10 g of polyethylene glycol is molten and dissolved in 75 ml of dichloromethane. The latter solution is added to the former and then there is added 2.5 g of magnesium octadecanoate, 5 g of polyvinylpyrrolidone and 30 ml of concentrated color suspension and the whole is homogenated. The tablet cores are coated with the thus obtained mixture in a coating apparatus.

### Example 14: Antiviral spectrum:

Because of the increasing emergence of drug resistant HIV strains, the present compounds were tested for their potency against clinically isolated HIV strains harboring several mutations. These mutations are associated with resistance to reverse transcriptase inhibitors and result in viruses that show various degrees of phenotypic cross-resistance to the currently commercially available drugs such as for instance AZT and delavirdine.

The antiviral activity of the compound of the present invention has been evaluated in the presence of wild type HIV and HIV mutants bearing mutations at the reverse transcriptase gene. The activity of the compounds is evaluated using a cellular assay and the residual activity is expressed in pEC₅₀ values. The columns IIIB and A-G in the table list the pEC₅₀ values against various strains IIIB, A- G.
Strain IIIB is wild type HIV-LAI strain
Strain A contains mutation Y181C in HIV reverse transcriptase,
Strain B contains mutation V106A in HIV reverse transcriptase,
Strain C contains mutation L100I in HIV reverse transcriptase,
Strain D contains mutation Y188L in HIV reverse transcriptase,
Strain E contains mutations L100I and K103N in HIV reverse transcriptase,
Strain F contains mutations K103N and Y181C in HIV reverse transcriptase.

| Comp. number | IIIB | A | B | C | D | E | F |
|---|---|---|---|---|---|---|---|
| 1 | 9.2 | 8.4 | 9.4 | 9.1 | 9.1 | 8.4 | 8.4 |
| 2 | 8.5 | 7.8 | 8.5 | 7.7 | 8.4 | 8.1 | 7.7 |
| 3 | 8.3 | 7.1 | 8.1 | 5.8 | 7.4 | 5.2 | 6.6 |

## Claims

1. A compound of formula 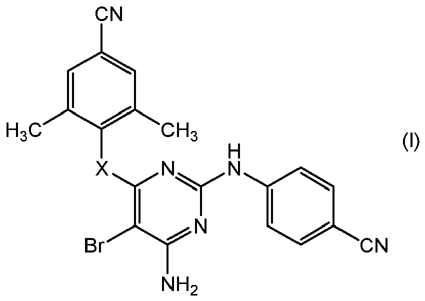 a pharmaceutically acceptable addition salt or a stereochemically isomeric form thereof, wherein
X is S, SO or SO₂.

2. A compound as claimed in claim 1 wherein X is S.

3. A compound as claimed in claim 1 or 2 for use as a medicine.

4. A pharmaceutical composition comprising a pharmaceutically acceptable carrier and as active ingredient a therapeutically effective amount of a compound as claimed in claim 1 or 2.

5. A process for preparing a composition according to claim 4 said process comprising intimately mixing the active ingredient and the carrier.

6. A process for preparing a compound of formula (I) as defined in claim 1 or 2, wherein:
(a) an intermediate of formula (II) or (IV) is reacted with an intermediate of formula (III) or (V), as outlined in the following reaction scheme, wherein X is as defined in claim 1 or 2 and W represents a suitable leaving group: 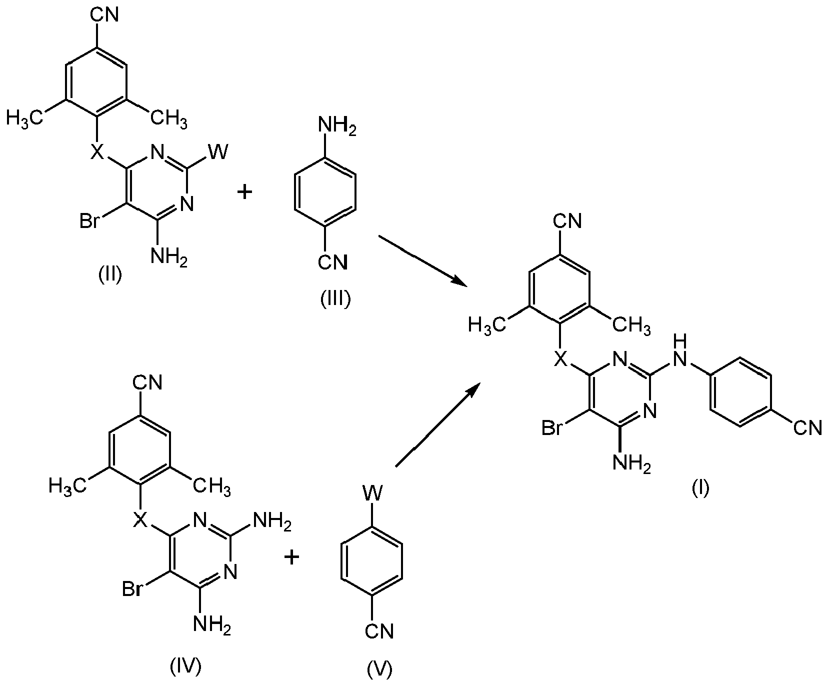
(b) a cyanophenyl derivative (VI) is reacted with a pyrimidine derivative (VII), or a cyanophenyl derivative (VIII) is reacted with with a pyrimidine derivative (IX), to prepare a compound of formula (I-a), which is a compound of formula (I) wherein X is S, as outlined in the following reaction scheme wherein X¹ is S, and W represents a suitable leaving group: 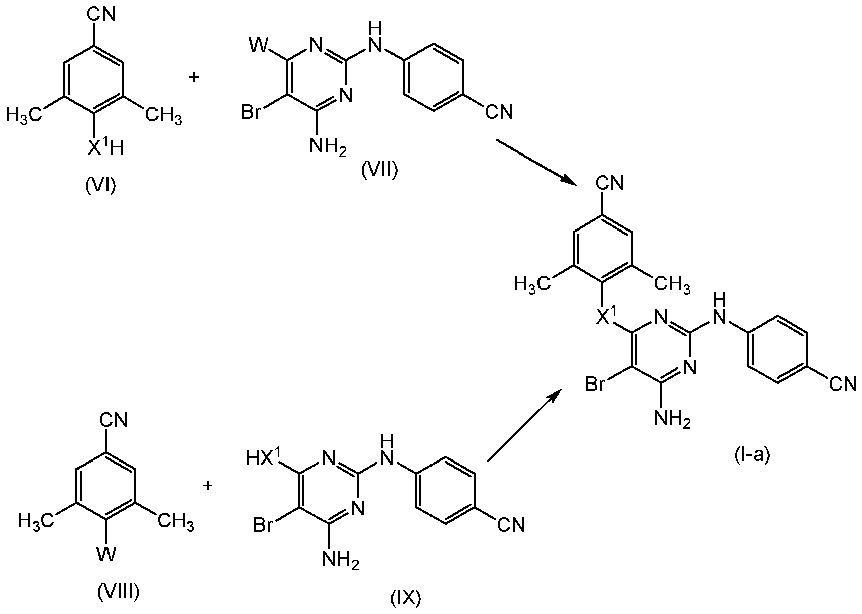
(c) a starting material (X) is brominated with free bromine, or with a bromine donor such as N-bromo succinimide, as outlined in the following scheme wherein X is as defined in claim 1 or 2: 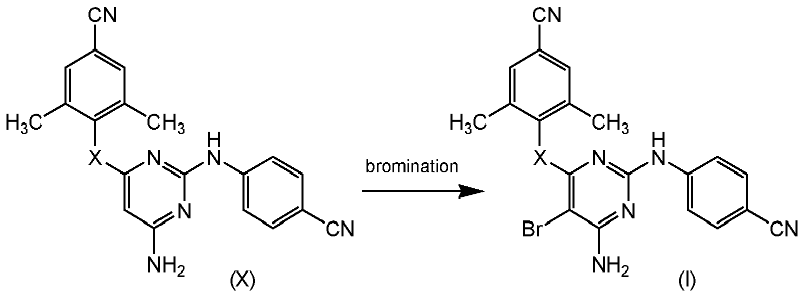
(d) a compound of formula (I) wherein X is S, is converted to the corresponding compound of formula (I) wherein X is SO or SO₂ by an oxidation reaction, such as with a suitable peroxide compound;
(e) a compound of formula (I) is converted into its pharmaceutically acceptable acid addition salt-form by treatment with an appropriate acid and vice versa a pharmaceutically acceptable acid addition salt-form of a compound of formula (I) is converted into the free base form by treatment of the salt with an appropriate base.
